Europäisches Patentamt

European Patent Office

Office européen des brevets

(19)

(11) Publication number: **0 173 189**
A2

(12) **EUROPEAN PATENT APPLICATION**

(21) Application number: **85110301.0**

(22) Date of filing: **17.08.85**

(51) Int. Cl.⁴: **B 01 J 23/20,** B 01 J 27/16

(30) Priority: **21.08.84 JP 173974/84**

(43) Date of publication of application: **05.03.86**
**Bulletin 86/10**

(84) Designated Contracting States: **AT BE CH DE FR GB IT**
**LI LU NL SE**

(71) Applicant: **CBMM Internacional Ltda., Rua Padre Joao**
**Manoe, 923, Sao Paulo Estado de Sao Paulo (BR)**

(72) Inventor: **Okazaki, Susumu, Prof.,**
**1973-60 Sakado-Machi, Mito Ibaragi (JP)**
Inventor: **Iizuka, Tokio, Dr., Hokkaido Sapporo-shi,**
**Toyohira-ku Nakano-shima no. 1-7 (JP)**
Inventor: **Kado, Satoshi, Kanagawa-ken Fujisawa-shi,**
**Miroku-ji, no. 3-26-1 (JP)**

(74) Representative: **Patentanwälte Wenzel & Kalkoff,**
**Grubes Allee 26 Postfach 730466,**
**D-2000 Hamburg 73 (DE)**

(54) **Solid acid catalyst in particular for accelerating chemical reactions.**

(57) A solid acid catalyst comprises essentially hydrated niobium oxide containing a phosphoric acid particularly on its surface in an amount sufficient to increase surface acidity, to inhibit crystallization of the niobium oxide and to retard loss in catalytic activity following exposure to high temperature. The catalyst can be prepared by treating hydrated niobium oxide ($Nb_2O_5 \cdot xH_2O$) or an anhydride thereof with a phosphoric acid. The catalyst is particularly useful in the hydration of ethylene to form ethanol.

14. August 1985

CBMM Internacional Ltda., Rua Padre Joao Manoel, 923
Sao Paulo - Estado de Sao Paulo, Brazil

Solid acid catalyst in particular for accelerating chemical
reactions

The invention relates to a solid acid catalyst in particular for accelerating chemical reactions such as the hydration of ethylene, alkylation or the like, its method of
preparation and its use in preparing an alcohol by hydration as well as a chemical compound by a process involving
the presence of water vapour.

Hitherto in chemical industries there exist several processes making use of sulphuric acid or aluminium chloride as
catalysts. Nevertheless, there are many problems inherent
in the use of these catalysts such as the separation and
recovery thereof, the treatment of the water used, the
corrosion of the installations and equipment, the formation
of by-products, the colouring of products, etc.

In the case of aluminium chloride there is also the problem
that high quantities of the catalyst are consumed.

Therefore, there is the demand to substitute these mineral
acids such as sulphuric acid, phosphoric acid, etc., by a
catalyst consisting of solid acids.

However, practically there is no corresponding catalyst
consisting of a solid acid which might be used in the
presence of water, except for some of the ion exchanging resins. However, these resins have certain deficiencies regarding their price, their facility of disaggregation, besides their limited range of application.

Lately, products of fluoric resin are being discussed, such as Nafion type H, having a higher acidity than that of the ion exchanging resins, but even so it shows insufficiency as to the acidity, besides being extremely costly.

Recently there has been proposed the production of a catalyst starting from hydrated niobium oxide (niobic acid) and submitting it to water washing, followed by a heat treatment at low temperature, or otherwise submitting such hydrated niobium oxide to a treatment with sulphuric acid or with hydrofluoric acid, followed by a heat treatment at low temperature, thus obtaining the hydrated niobium oxide or acid-treated hydrated niobium oxide, respectively, which form a solid acid catalyst.

However, even in this catalyst there occur deficiencies due to the occurrence of crystallization at high temperatures, for example, over 400 ºC, and particularly over 500 ºC, whereby the catalytic activity is deteriorated and the use thereof in reactions to be carried out at high temperatures is not possible.

On the other hand, if such a catalyst has lost its catalytic activity and if in the reactivation thereof a temperature exceeding 400 ºC should be required, such a temperature could hardly be reached to provide the reactivation in practical terms. In consequence thereof the utilization of this hydrated niobium oxide catalyst as an industrial catalyst would be most difficult.

It is therefore a main and essential task of the present invention to resolve the above problems by suggesting a catalyst which has an improved surface acidity and in which the crystallization of the acid in question is inhibited simultaneously.

According to the present invention this task is solved in that the catalyst comprises as effective component niobic acid (hydrated niobium oxide) treated with phosphoric acid. In other terms the solid acid catalyst is obtained by treating hydrated niobium oxide ($Nb_2O_5.nH_2O$) with phosphoric acid thereby enhancing its surface acidity and simultaneously preventing its crystallization which would decrease the catalytic activity. This acid-treated niobic acid can be used for hydration of ethylene, alkylation, etc., i.e. chemical reactions that can be accelerated by an acid catalyst. The essence of the invention thus resides in the treatment of the niobic acid with phosphoric acid to give it a higher acidity on its surface, at the same time inhibiting the crystallization of the acid in question.

According to a particular aspect of the invention the effective component consists of phosphoric acid impregnated niobic acid, which is advantageously effective in the presence of water vapour.

Expediently the solid acid catalyst is prepared by washing niobic acid (hydrated niobium oxide) with water, then impregnating it with phosphoric acid, whereafter the resulting product is dried, pulverised to convenient size and activated by heat treatment at a temperature of about 200 oC to about 600 oC, preferably of approximately 300 oC to about 600 oC.

The solid acid catalyst thus obtained may be used with preference in the preparation of an alcohol, preferably ethanol, by hydration of an olefin, preferably ethylene, which is reacted with water vapour in the presence of a catalytically effective amount of such catalyst. Said water vapour affording process may be a dehydration, a hydration, an esterification process or the like.

The invention will be more readily understood from the following detailed description of the preferred embodiments and from the accompanying drawings in which:

Fig. 1    shows the relationship between the surface acidity strengths of niobic acid treated with phosphoric acid of 0.5 mol/l at different temperatures;

Fig. 2    shows the relationship between the surface acidity strengths of niobic acid treated with phosphoric acid of 1.0 mol/l at different temperatures;

Fig. 3    shows the influence of the acid-treatment of hydrated niobium oxide on its surface acid amount with the temperature, considering $H_0 \leqslant -5.6$;

Fig. 4    shows the catalytic activity of niobic acid treated with phosphoric acid of 0.5 mol/l for ethylene hydration; and

Fig. 5    shows the catalytic activity of niobic acid treated with phosphoric acid of 2 mol/l for ethylene hydration.

The inventors have treated niobic acid ($Nb_2O_5 \cdot nH_2O$) with diluted phosphoric acid, in the form of solutions of 0,5 mol/l and 1,0 mol/l, and then submitted it to a thermic treatment at temperatures of 200 °C, 400 °C, 600 °C and

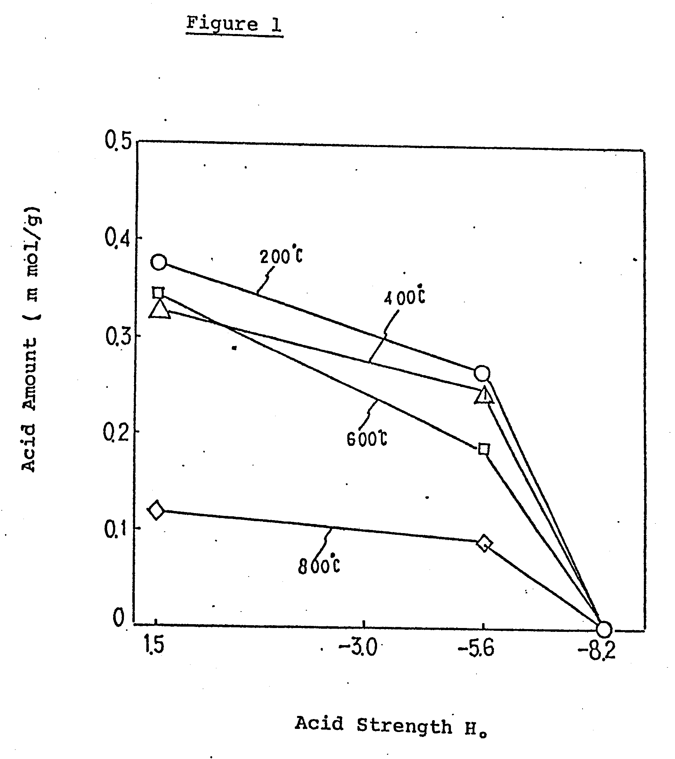

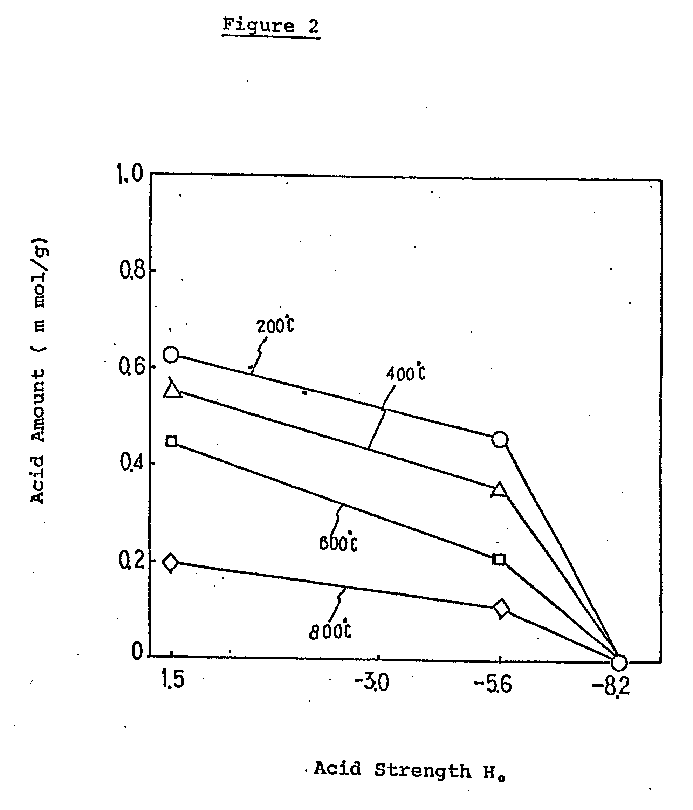

800 ºC, successively.  They obtained a niobic acid with a tendency to a relatively high surface acidity by the value of $H_0 \leqslant -5,6$, as indicated in Fig. 1 and 2.  Furthermore it has been clarified that the quantity of the superficial acid maintained at high temperatures, exceeding 600 ºC, does not practically depend on the concentration of the phosphoric acid.

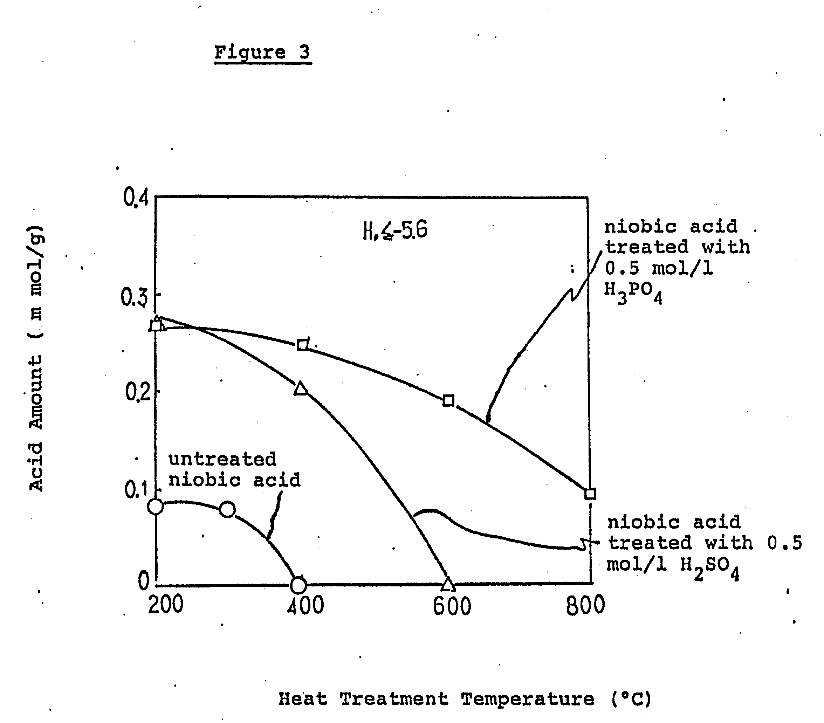

As shown in Fig. 3, which compares non-treated niobic acid, niobic acid treated with sulphuric acid, and niobic acid treated with phosphoric acid, the niobic acid treated with phosphoric acid keeps a sufficiently strong and exact surface acidity even when heated at a temperature exceeding 500 ºC, or even up to 800 ºC.

It is supposed that this phenomenon is due to a condensation of the phosphoric acid, forming a non-volatile polyphosphoric acid on the surface and in the vicinity of the surface of the niobic acid treated with phosphoric acid.  It is also supposed that the contents of the phosphoric acid inhibits the crystallization of the niobic acid at high temperatures, at the sime time producing the formation of a strong acidity, and also contributing to its intensification.  Furthermore, the fact that the capacity of preserving the strong acidity at high temperatures does not depend on the concentration of the phosphoric acid used for the treatment is demonstrated by the formation of a polyphosphoric acid film or the like, sufficiently capable of covering the niobic acid, even if the latter has been treated with relatively highly diluted 0,5 mol/1 phosphoric acid.

On the other hand, it becomes clear that this acid characteristic is also resistant to the poisoning of the catalyst by water.  Furthermore, this catalyst consisting of solid acid shows an excellent catalytic activity, especially in the hydration of olefines, and at the same time it has a high catalytic capacity as a catalyst of acid-catalytic reactions involving the presence of the water (hydration, esterification, Beckman rearrangement, etc.).

As an example, an explanation will be given below about the catalytic activities in the hydration of the ethylene.

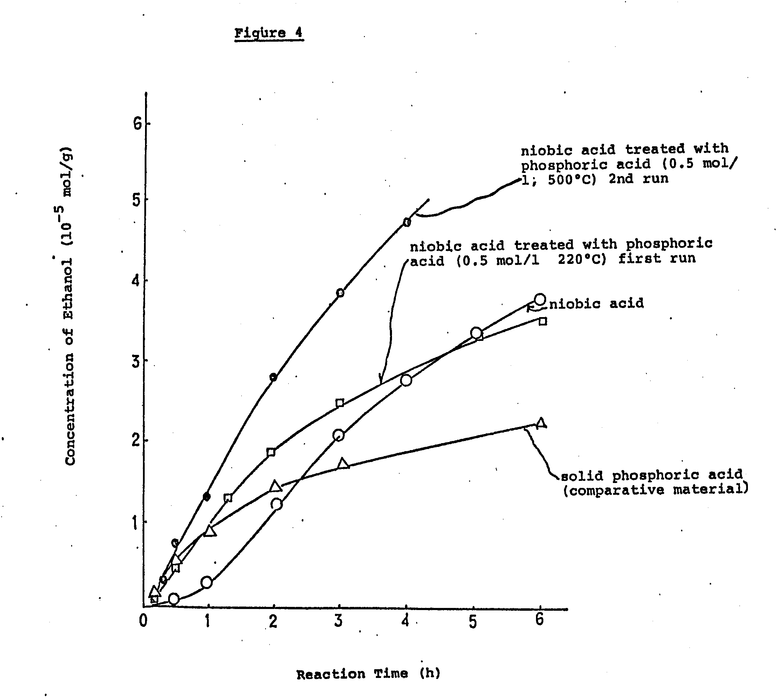

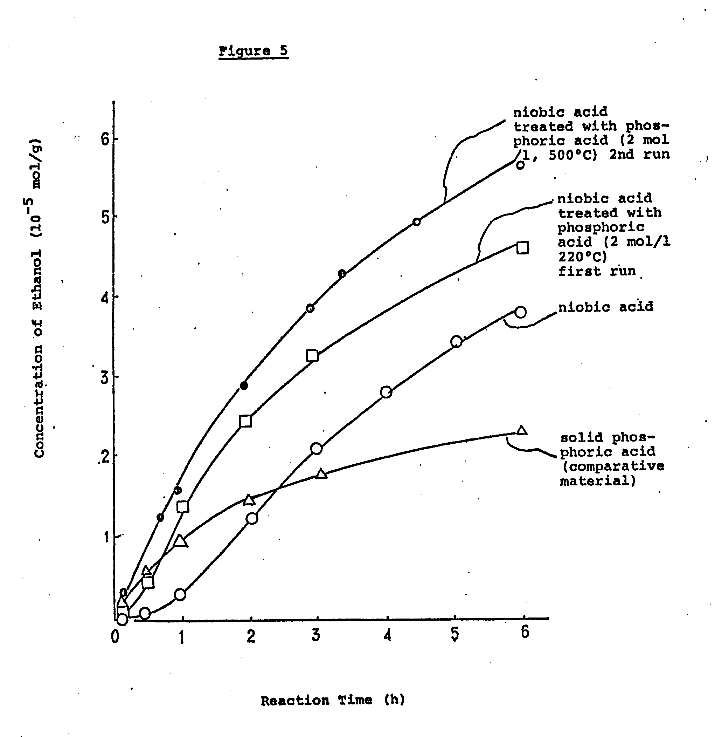

In Fig. 4 and 5 there can be found the results of the calculation concerning the catalytic activity of niobic acid treated with 0,5 mol/l and 2 mol/l of phosphoric acid in a hydration of ethylene, by making use of an equipment for processing gaseous phase reaction in a circulation system.

The figures also indicate the activities of non-treated niobic acid and of the solid phosphoric acid under the same reaction conditions.

As clearly shown in the figures, non-treated niobic acid

shows a stronger catalytic activity than that of the solid phosphoric acid. Nevertheless, at the beginning of the reaction the catalytic activity is quite low, demonstrating the existence of the introduction period. In relation to the latter, the niobic acid treated with phosphoric acid indicates a high activity right from the beginning of the reaction. The result to which special attention should be given is that, when the reaction starts again by expelling the gas heated at a high temperature of 500 ºC, the catalytic activity is increased even more.

This is in contrast to non-treated niobic acid, the catalytic effect of which, even when reaching the exhaustion of gas at a temperature of 300 ºC to 400 ºC, remains markedly low.

By using the results obtained from the calculation of the specific surface through test procedures by X-ray diffraction of the pulverised sample, mass differential thermic analysis, and by the method of nitrogen absorption, etc., one can see that, while rapid crystallization occurs in the non-treated niobic acid close to 550 ºC, when phosphoric acid is added the niobic acid is controlled in such a manner that merely a trace of crystallization shows up, even if the temperature is increased over 600 ºC.

The following data can be obtained about the influence of concentration of the phosphoric acid used for the treatment, namely:  In the first reaction, the niobic acid treated with 2 mol/l of phosphoric acid, as shown in Fig. 5, demonstrates a higher activity in relation to the niobic acid treated with 0,5 mol/l of phosphoric acid as shown in Fig. 4.  However, after heating and exhaustion at 500 ºC in the second reaction, the catalytic activity of treated niobic acid adopts  a constant value, independent of the concentration of the phosphoric acid.  It is supposed that this is due to the level of surface acidity of the niobic acid which is $H_0 \leqslant -5,6$, after treatment at a high temperature, as shown in Fig. 1 and 2, thus remaining practically constant independent of the concentration of the phosphoric acid.  On the other hand, as the catalytic activity is markedly superior to that of the solid phosphoric acid, the effect of the treatment which phosphoric acid as observed in the niobic acid is not merely due to the contents of the phosphoric acid becoming condensed and associated, but also due to the fact of being fixed to the niobic acid surface and becoming spread and widely expanded.

Examples:

10 g of niobic acid ($Nb_2O_5 \cdot nH_2O$) are immerged into 30 ml of an aqueous solution of phosphoric acid ($H_3PO_4$) 0,5 or
1,0 mol/l, and left still for 48 hours.  The drying is made
by outside air at 120 oC, and after hardening it is ground
to grains smaller than 100 mesh and used as a catalyzer
for ethanol synthesis through ethylene hydration.

Each catalyst has been heated through exhaustion (hot air
flux) during 2 hours at an established temperature inside
the tubular reactor and subsequently used for the reaction.

The reactor used for the experiment is an air-tight installation with a system for reaction by circulation of a
200 ml capacity.  Immediately following the tubular reactor,
a hydration equipment is installed and to it, by placing
5 ml of water and immerging it into a tank with constant
temperature, a water steam is fed at constant pressure.
To prevent the water condensation the whole reaction
system is kept at a certain temperature, heated by a coil
heater.

The product formed is dissolved in the hydration equipment and a certain quantity of sample is removed and analysed by chromatography in the gaseous phase.  The reaction conditions are as follows:  quantity of catalyst,
0,8 to 0,9 g; partial pressure of ethylene, 400 Torr;
partial pressure of water steam, 55 Torr; reaction temperature, 220 oC.

The comparative results of the synthesis reaction are indicated hereinafter (Table 1), with the present catalyst,
with the results of the reaction, by making use of a
catalyst treated with sulphuric acid and phosphoric acid.

Table 1.  Reaction of hydration of ethylene.

| Catalyst | Number of days for reaction | Temperature of exhaustion flux (°C) | Speed of formation of ethanol (g-lh-l) | Maximum strength of acid |
|---|---|---|---|---|
| $Nb_2O_2$ | 1 | 220 | 0.83 | -8.2 |
| $Nb_2O_5-SO_4^{2-}$ (treated with $H_2SO_4$ 0,1 mol/l) | 1 | 220 | 1.33 | -8.2 |
| $Nb_2O_5-F^-$ (treated with HF 13% in weight) | 1 | 220 | 1.11 | -8.2 |
| Polyphosphoric acid | 1 | 220* | 0.70 | |
| $Nb_2O_5-PO_4^{3-}$ (treated with $H_3PO_4$ 2 mol/l) | 1 | 220 | 1.31 | |
| " | 2 | 500 | 1.53 | |
| $Nb_2O_5-PO_4^{3-}$ (treated with $H_3PO_4$ 0,5 mol/l) | 1 | 220 | 0.99 | |
| " | 2 | 500 | 1.55 | |

* After reaching 220 °C, the time of exhaustion of flux is 3 minutes.  The remaining time is 2 hours.

0173189

After performing the reaction with untreated niobic acid during 3 hours, at 200 °C, and using it again for the reaction an increase of activity occurs. However, when the catalyst is used at a temperature exceeding 400 °C, the catalytic activity rapidly decreases to zero. If the catalyst is treated with hydrofluoric acid or with sulphuric acid, there is an increase in the catalytic activity, but after performing the reaction at a high temperature and also when the catalyst is used a a high temperature, its catalytic activity disappears.

On the other hand, by making use of solid phosphoric acid which is currently used on an industrial scale for the hydration of ethylene, under the same above mentioned conditions, though a high activity can be observed at the beginning of the reaction, after 2 hours' exhaustion, the result of the re-utilisation to serve as catalyst in the new reaction is that its catalytic activity is greatly reduced.

It is supposed that this is due to the fact that the contents of phosphoric acid may have evaporated during the reaction. With reference thereto, in the niobic acid treated with phosphoric acid in comparison with the non-treated niobic acid, or treated with other acids, a high catalytic activity occurs from the beginning of the reaction, and when the catalyst is reactivated at a high temperature one can see that its catalytic activity becomes even higher.

As explained above, in the catalyst according to the present invention the crystallization does not develop even at high temperatures, for example, over 500 °C, and there is no degeneration of the catalytic activity, either. Therefore, it is possible to use this catalyst success-

0173189

fully in reactions at high temperatures.  Even extremely
high temperatures can be adopted, i.e., over 400 ºC, to
reactivate the catalytic power in case this has been lost,
which demonstrates the high applicability as a catalyst
for industrial purposes.

Patent   Claims :

1. A solid acid catalyst in particular for accelerating chemical reactions such as the hydration of ethylene, alkylation etc., c h a r a c t e r i s e d   i n that the catalyst comprises as effective component niobic acid (hydrated niobium oxide) treated with phosphoric acid.

2. A catalyst as claimed in claim 1, c h a r a c t e - r i s e d   i n   that the effective component consists of niobic acid impregnated with phosphoric acid.

3. A catalyst as claimed in claim 1 or 2, c h a r a c - t e r i s e d   i n   that it is effective in the presence of water vapour.

4. A method of preparing a solid acid catalyst by submitting niobic acid (hydrated niobium oxide) to an acid treatment, c h a r a c t e r i s e d   b y the steps of

   a) washing niobic acid with water;

   b) impregnating the washed niobic acid with phosphoric acid;

   c) drying the resulting product;

   d) pulverising the dried product to convenient size;

   e) activating the pulverised product by heat treatment at a temperature of about 200 ºC to about 600 ºC.

0173189
14.08.1985

5. A method as claimed in claim 4, c h a r a c t e - r i s e d   i n   that the activation is performed by heat treatment at a temperature of about 300 ºC to about 600 ºC.

6. A method of preparing an alcohol by hydration of an olefin, c h a r a c t e r i s e d   i n   that the olefin is reacted with water vapour in the presence of a catalytically effective amount of the solid acid catalyst as prepared according to claim 4 or 5.

7. A method as claimed in claim 6, c h a r a c t e - r i s e d   i n   that the olefin is ethylene and the alcohol is ethanol.

8. A method for preparing a chemical compound by a process involving the presence of water vapour such as dehydration, hydration, esterification or the like, c h a r a c t e r i s e d   i n   that the process is performed in the presence of the solid acid catalyst as prepared according to claim 4 or 5.

Figure 1

Figure 2

Acid Strength H₀

# Figure 3

Axis label (y): Acid Amount ( m mol/g )

Axis label (x): Heat Treatment Temperature (°C)

In-figure labels: H,≤-5.6; niobic acid treated with 0.5 mol/l $H_3PO_4$; untreated niobic acid; niobic acid treated with 0.5 mol/l $H_2SO_4$

Figure 4

niobic acid treated with phosphoric acid (0.5 mol/1, 500°C) 2nd run

niobic acid treated with phosphoric acid (0.5 mol/1 220°C) first run

niobic acid

solid phosphoric acid (comparative material)

Reaction Time (h)

Concentration of Ethanol ($10^{-5}$ mol/g)

Figure 5